# EUROPEAN PATENT APPLICATION

(11) **EP 1 952 808 A2**
(43) Date of publication of application: **06.08.2008**
(21) Application number: 08100323.8
(22) Date of filing: 17.11.2004
(51) Int. Cl.: A61K 31/09, A61K 47/14, A61K 47/24, A61K 47/44

(54) **Composition in the form of a spray comprising calcitriol**

(30) Priority: 21.11.2003 FR 0313660; 04.02.2004 US 541245 P
(62) Divisional of application: 04803732.9
(71) Applicant: GALDERMA RESEARCH & DEVELOPMENT, 06410 Biot (FR)
(72) Inventor: Pitre, Franck, 77600 Bussy Saint Georges (FR); Fredon, Laurent, 06330 Roquefort les Pins (FR); Mallard, Claire, 06250 Mougins (FR)
(74) Representative: Allab, Myriam

(57) **Abstract**

The invention relates to a composition in the form of a spray comprising a pharmaceutical active agent, at least one volatile silicone and a nonvolatile oily phase in a physiologically acceptable medium, to the process for preparing it and to its use in cosmetics and in dermatology.

## Description

The invention relates to a composition comprising a pharmaceutical active agent, at least one volatile silicone and a nonvolatile oily phase in a physiologically acceptable medium, to the process for preparing it and to its use in cosmetics and in dermatology, the composition making it possible to obtain good penetration of the active agent through the layers of the skin.

In the field of dermatology and of the formulation of pharmaceutical compositions, those skilled in the art are led to search for compositions which make it possible to release the active agent and to promote its penetration through the layers of the skin in order to improve its effectiveness. The product should also show good cosmeticity and preferably be nonirritant.

There are currently many topical compositions comprising an active agent and making it possible to promote penetration thereof into the skin by means of the presence in particular of a high content of pro-penetrating glycol. These compositions are formulated in the form of emulsions with a high content of fatty phase, which are commonly called "lipocreams", in the form of anhydrous compositions which are called "ointments", in the form of fluid compositions with a high content of volatile solvents, such as ethanol or isopropanol, intended for application to the scalp, also called "hair lotions", or else in the form of viscous O/W emulsions, which are also called "O/W creams".

O/W creams comprising a corticoid and a high percentage of propylene glycol (47.5%), sold under the trade mark TEMOVATE® by the company GLAXOSMITHKLINE, are, for example, known. The stabilizing of a formulation comprising such a percentage of glycol makes it necessary to use, in the emulsion, emulsifiers and stabilizers of the glyceryl stearate or PEG 100 stearate type, or alternatively stabilizers or consistency factors of the white wax or cetostearyl alcohol type, which result in the formation of a viscous cream, that is to say a cream with a viscosity greater than 10 Pa.s (10 000 centipoises, measured with a Brookfield model LVDV II + mobile No. 4 device, at a rate of 30 rpm for 30 seconds and at a temperature of 25°C ± 3°C). This viscosity therefore makes the product difficult to apply. These compositions therefore show, firstly, poor cosmetic acceptability due to their viscosity and, secondly, risks of intolerance caused by the presence of high proportions of glycol. Those skilled in the art therefore wish to improve these parameters, by virtue of the present invention.

In order to facilitate the application of topical compositions comprising a high percentage of pro-penetrating glycol, the applicant has produced, and protected by means of application EP 832647, a lotion, which is a stable formulation of O/W emulsion type, and the viscosity of which is intermediate between hair lotions which are too fluid and have too limited a use, and O/W creams which are too viscous and have a greasy and sticky side to them, while at the same time conserving the pro-penetrating properties of the glycol. These formulae effectively show good penetration of the active agent, but still comprise a high percentage of glycol which can therefore induce a sticky effect or problems of tolerance resulting in moderate acceptability of the product by the patient.

Formulations containing silicone compounds which result in compositions which are pleasant to use are, moreover, known to those skilled in the art. Thus, in US patent 6,538,039, a novel formulation of active agent for transdermal administration has been developed, comprising silicone compounds in order to deposit a film at the surface of the skin. In that application also, the transdermal passage is facilitated by the obligatory presence of absorption promoters, namely, among other compounds mentioned, glycols.

In patent application EP 0966972, the compositions described can be formulated in the form of a spray and comprise an active compound, a silicone gum and a pharmaceutically acceptable excipient. The problem that the invention described in EP 0966972 proposes to solve is that of depositing a substantive film at the surface of the skin, which problem is solved by means of the presence of the silicone gum.

The problem that the present invention here proposes to solve is that of designing a composition for improving the penetration of the pharmaceutical active agent, and its rapidity of penetration over time, in order to improve its therapeutic efficacy, while at the same time avoiding the presence of a high content of glycol. The composition according to the invention should also be easy to use and show a cosmeticity which is acceptable for application to all the regions of the body which may be affected by the pathology.

The two applications EP 0966972 and US 6,538,039 represent the prior art closest to the present invention, given the composition of the formulations described. However, on reading this prior art, there is nothing which could prompt those skilled in the art to choose the composition according to the invention in order to obtain good penetration of the active agent incorporated, into the layers of the skin.

In fact, the applicant has found, surprisingly, that the composition comprising, in a pharmaceutically acceptable vehicle:
a) a therapeutically effective amount of a pharmaceutical active agent,
b) at least one volatile silicone,
c) a nonvolatile oily phase,
results in an improvement in penetration of the active agent.

The composition of the present invention, while allowing good penetration of the active principles, also shows very good acceptability and tolerance among patients, as described in Examples 8 and 9 of the present invention. It is therefore found that the composition according to the invention is particularly suitable for the treatment of dermatological conditions, and more particularly very suitable for the treatment of psoriasis.

The invention relates more particularly to a composition comprising, in a pharmaceutically acceptable vehicle:
a) a therapeutically effective amount of a pharmaceutical active agent,
b) at least one volatile silicone,
c) a nonvolatile oily phase,
characterized in that the pharmaceutical active agent is a compound derived from vitamin D.

The term "compound derived from vitamin D" is intended to mean compounds which exhibit biological properties similar to those of vitamin D, in particular the properties of trans-activation of vitamin D response elements (VDREs), such as agonist or antagonist activity with respect to receptors for vitamin D or for its derivatives. Compounds derived from vitamin D that are useful according to the invention thus comprise structural analogues, for example bioaromatic analogues. The expression "vitamins D or their derivatives" is intended to mean, for example, the derivatives of vitamin D₂ or D₃, and in particular 1,25-dihydroxy vitamin D₃ (calcitriol).

Among the pharmaceutical active agents derived from vitamin D which can be used according to the invention, mention may be made, by way of non limiting examples, of the compounds described in patent applications EP 1124779, EP 1235824, EP 1235777, WO 02/94754 and WO 03/050067.

Preferably, the vitamin D derivatives according to the invention are the compounds described in patent FR 2785284, incorporated herein by way of reference. They are compounds which are structural analogues of vitamin D and which show selective activity on cell proliferation and differentiation without being hypercalcian-inducing in nature.

These compounds can be represented by general formula (I) below: 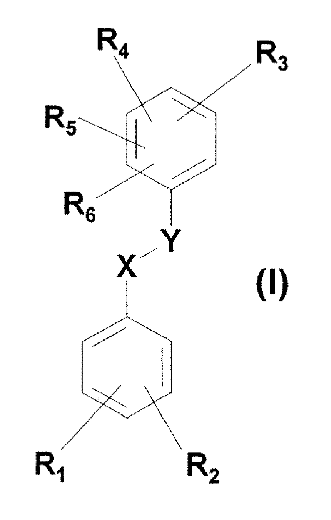 in which:
- R₁ represents a hydrogen atom, a methyl radical or a radical - (CH₂)ₙ-OR₇,
- R₂ represents a radical - (CH₂)ₙ-OR₈,
   n, R₇ and R₈ having the meanings given below,
- X-Y represents a bond chosen from the bonds of formulae (a) to (d) below which can be read from left to right or vice versa: 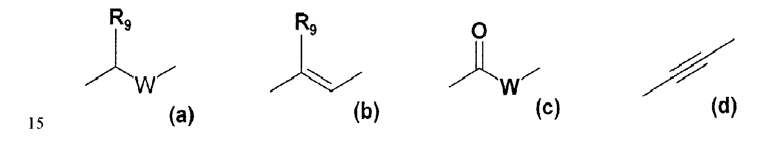 R₉ and W having the meanings given below,
- R₃ represents the chain of vitamin D₂ or vitamin D₃, 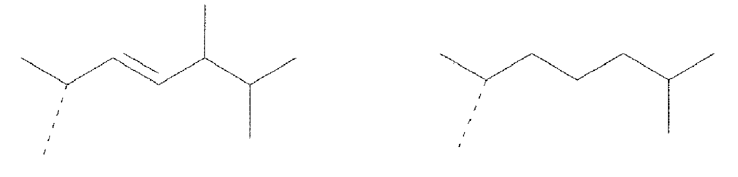 the dashed lines represent the bond linking the chain to the benzene ring represented in figure (I),
   or R₃ represents a chain having from 4 to 8 carbon atoms substituted with one or more hydroxyl groups, it being possible for the hydroxyl groups to be protected in acetoxy, methoxy or ethoxy, trimethylsilyloxy, tert-butyldimethylsilyloxy or tetrahydropyranyloxy form, and optionally, in addition:
- substituted with one or more lower alkyl or cycloalkyl groups and/or
- substituted with one or more halogen atoms and/or
- substituted with one or more groups CF₃ and/or
- in which one or more carbon atoms of the chain are replaced with one or more oxygen, sulphur or nitrogen atoms, it being possible for the nitrogen atoms to be optionally substituted with lower alkyl radicals and/or
- in which one or more single bonds of the chain are replaced with one or more double and/or triple bonds,
- R₃ being positioned, on the benzene ring, in the position *para* or *meta* to the X-Y bond,
- R₄, R₅ and R₆, two of which may be identical or different, represent a hydrogen atom, a lower alkyl radical, a halogen atom, a radical -OR₁₀ or a polyether radical,
   R₁₀ having the meaning given below,
- n being 0, 1 or 2,
- R₇ and R₈, which may be identical or different, represent a hydrogen atom, an acetyl radical, a trimethylsilyl radical, a tert-butyldimethylsilyl radical or a tetrahydropyranyl radical,
- R₉ represents a hydrogen atom or a lower alkyl radical,
- W represents an oxygen or sulphur atom, a radical
- CH₂- or a radical -NH- which can optionally be substituted with a lower alkyl radical,
- R₁₀ represents a hydrogen atom or a lower alkyl radical,
and also the optical and geometrical isomers of said compounds of formula (I), and the salts thereof when X-Y represent a bond of formula (a) and W represents a radical -NH- optionally substituted with a lower alkyl radical.

Among the compounds of formula (I) that are useful in the compositions of the present invention, mention may in particular be made of the following:
1. 6-[3-(3,4-bishydroxymethylbenzyloxy)phenyl]-2-methylhepta-3,5-dien-2-ol,
2. 7-[3-(3,4-bishydroxymethylphenoxymethyl)phenyl]-3-ethyloctan-3-ol,
3. 7-{3-[2-(3,4-bishydroxymethylphenyl)ethyl]phenyl}-3-ethylocta-4,6-dien-3-ol,
4. 6-{3-[2-(3,4-bishydroxymethylphenyl)ethyl]phenyl}-2-methylhepta-3,5-dien-2-ol,
5. 7-{3-[2-(3,4-bishydroxymethylphenyl)vinyl]phenyl}-3-ethylocta-4,6-dien-3-ol,
6. 7-[3-(3,4-bishydroxymethylbenzyloxy)phenyl]-3-ethyl-3-octanol,
7. 4E,6E)-7-[3-(3,4-bishydroxymethylbenzyloxy)-phenyl]-3-ethylocta-4,6-dien-3-ol,
8. (4E,6E)-7-[3-(3,4-bishydroxymethylbenzyloxy)-phenyl]-3-ethylnona-4,6-dien-3-ol,
9. (E)-7-[3-(3,4-bishydroxymethylbenzyloxy)phenyl]-3-ethyloct-4-en-3-ol,
10. (E)-7-[3-(3,4-bishydroxymethylbenzyloxy)phenyl]-3-ethyloct-6-en-3-ol,
11. (E)-7-[3-(3,4-bishydroxymethylbenzyloxy)phenyl]-3-ethyloct-6-en-4-yn-3-ol,
12. (4E,6E)-7-[3-(3,4-bishydroxymethylphenoxymethyl)-phenyl]-3-ethylocta-4,6-dien-3-ol,
13. (E)-7-[3-(3,4-bishydroxymethylphenoxymethyl)-phenyl]-3-ethylnon-6-en-3-ol,
14. (E)-7-{3-[(3,4-bishydroxymethylbenzyl)methylamino]phenyl}-3-ethyloct-6-en-3-ol,
15. 7-[3-(3,4-bishydroxymethylbenzyloxy)phenyl]-3-ethyl-7-methyloctan-3-ol,
more preferably, the pharmaceutical active agent incorporated into the composition according to the invention is (4E,6E)-7-[3-(3,4-bishydroxymethylbenzyloxy)phenyl]-3-ethylnona-4,6-dien-3-ol.

Advantageously, the composition according to the invention comprises between 0.0001 and 20% by weight, relative to the total weight of the composition, of an active agent, preferably between 0.025 and 15% by weight, and more preferably between 0.01 and 5% by weight.

Of course, the amount of active agent in the composition according to the invention will depend on the active agent under consideration.

The composition according to the invention will preferably comprise an active agent derived from vitamin D at a concentration of less than 2% by weight of active agent, preferably of between 0.025 and 0.5% by weight. The preferred pharmaceutical active agent according to the invention is (4E,6E)-7-[3-(3,4-bishydroxymethylbenzyloxy)phenyl]-3-ethylnona-4,6-dien-3-ol used at a concentration of 0.3% by weight.

The active agents which can be used according to the invention may be used alone or in combination.

According to the invention, the term "volatile silicone" is intended to mean polyorganosiloxane compounds, which may be cyclic or linear, having a measurable pressure under ambient conditions. The cyclic volatile silicones according to the invention are polydimethylcyclosiloxanes, i.e. compounds of formula: 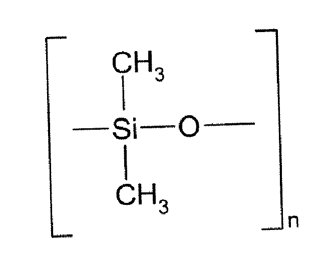 with n being, on average, between 3 and 6, and preferably n=4 or n=5, generally known as cyclomethicones. The linear volatile silicones according to the invention are linear polysiloxanes such as hexamethyldisiloxane or low molecular weight dimethicones. The linear volatile silicones generally have a viscosity of less than approximately 5 centistokes at 25°Celsius, whereas the cyclic volatile silicones have a viscosity of less than approximately 10 centistokes at 25°Celsius.

Preferred volatile silicones according to the invention are the linear siloxanes, and more preferably hexamethyldisiloxane. By way of example, mention may be made of the product sold by the company DOW CORNING, DC Fluid 0.65cSt.

Advantageously, the composition according to the invention comprises between 25 and 95% by weight, relative to the total weight of the composition, of the volatile silicone, and preferably between 40 and 80% by weight, and more preferably between 55 and 65% by weight.

According to the invention, the term "nonvolatile oily phase" is intended to mean a variety of nonvolatile oil suitable for a pharmaceutical or cosmetic composition. The nonvolatile oils generally have a viscosity of greater than approximately 10 centipoises at 25°C, and can reach a viscosity ranging up to 1 000 000 centipoises at 25°C. The nonvolatile oily phase can be made up of a large variety of synthetic or natural, silicone or organic oils, a nonexhaustive list of which is given by way of indication.

### (a) Esters

Examples of a nonvolatile oil which can be used according to the invention comprise esters of formula RCO-OR' with R and R', which may be identical or different, representing a linear or branched chain of an alkyl, alkenyl, alkoxycarbonylalkyl or alkoxycarbonyloxyalkyl containing from 1 to 25 carbon atoms, preferably from 4 to 20 carbon atoms. Examples of such esters include isotridecyl isononanoate, PEG-4 diheptanoate, isostearyl neopentanoate, tridecyl neopentanoate, cetyl octanoate, cetyl palmitate, cetyl ricinoleate, cetyl stearate, cetyl myristate, coco dicaprylate/caprate, decyl isostearate, isodecyl oleate, isodecyl neopentanoate, isohexyl neopentanoate, octyl palmitate, dioctyl malate, tridecyl octanoate, myristyl myristate and octododecanol.

### (b) Glyceryl esters of fatty acids

The oil may also comprise fatty esters of natural fatty acids, or triglycerides of animal or plant origin. Such examples include, castor oil, lanolin oil, triisocetyl citrate, triglycerides containing from 10 to 18 carbon atoms, caprylic/capric triglycerides, coconut oil, corn oil, cottonseed oil, flax oil, mink oil, olive oil, palm oil, illipe butter, rapeseed oil, soybean oil, sunflower oil, nut oil and equivalent.

### (c) Fatty acid glycerides

The oils which are also suitable are synthetic or semi-synthetic glyceryl esters, such as fatty acid mono-, di or triglycerides, which are modified natural oils or fats, for example glyceryl stearate, glyceryl dioleate, glyceryl distearate, glyceryl trioctanoate, glyceryl linoleate, glyceryl myristate, glyceryl isostearate, PEG castor oils, PEG glyceryl oleates, PEG glyceryl stearates, and equivalent.

### (d) Nonvolatile hydrocarbons

Nonvolatile hydrocarbons such as paraffins, isoparaffins, mineral oils, and equivalent are also very suitable for the composition according to the invention, as non-volatile nonpolar solvent.

### (e) Guerbet esters

Guerbet esters are esters resulting from the reaction of a Guerbet alcohol of general formula: 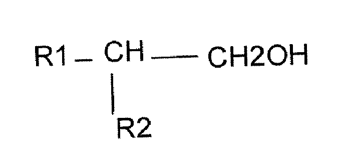 and a carboxylic acid of general formula R3-COOH or HOOC-R3-COOH,
R1 and R2, which may be identical or different, represent an alkyl containing from 4 to 20 carbon atoms, and R3 represents a substituted or unsubstituted fatty radical, such as a linear or branched, saturated or unsaturated alkyl or alkylene chain containing from 1 to 50 carbon atoms, a phenyl, which may be substituted with a halogen, a hydroxyl, a carboxyl, or an alkylcarbonylhydroxyl.

### (f) Silicone oils

The silicone oils which can be used according to the invention for making up the nonvolatile phase are polyorganosiloxane compounds having a measurable pressure under ambient conditions and a viscosity strictly greater than 10 centistokes and lower than 20 centistokes. The nonvolatile silicones which can be used according to the invention are the compounds of formula: 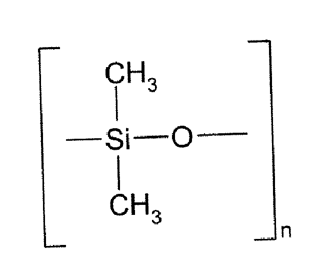 with n strictly greater than 6.

The preferred nonvolatile oily phase according to the invention is paraffin oil.

Advantageously, the composition according to the invention comprises between 1 and 50% by weight, relative to the total weight of the composition, of nonvolatile oily phase, preferably between 5 and 30% by weight, and more preferably between 7 and 15% by weight.

According to a preferred embodiment of a composition according to the invention, the composition also comprises a silicone gum. The applicant has, in fact, discovered, surprisingly, that a composition comprising a silicone gum in the concentrations defined hereinafter shows more rapid penetration of the active agent through the various layers of the skin.

The term "silicone gums" is intended to mean the silicone gums known to those skilled in the art, and in particular those described in patent application EP 0966972, incorporated herein by way of reference. According to this preferred embodiment of a composition according to the invention, the silicone gum is introduced at a concentration of between 0.001 and 3% by weight, preferably between 0.01 and 1% by weight. Dow Corning provides a commercial product sold under the name DC Silmogen Carrier, which is made up of 99% of hexamethyldisiloxane and 1% of silicone gum, which product may advantageously be used in one of the compositions according to the invention.

The pharmaceutically acceptable vehicle according to the invention should be chosen such that the advantageous properties intrinsically associated with the present invention are not, or are not substantially, altered by the envisaged addition. Preferably, the vehicle used according to the invention is chosen so as to be an agent which solubilizes the active agent. The active agent-solubilizing vehicle may be made up of a single excipient, such as a solvent, or of a mixture of excipients, such as those used for the formulation of an emulsion. By way of nonlimiting examples of excipients which may be used alone or as a mixture, mention may be made of water, solvents, diluents, and any excipient which can be used for the formulation of an emulsion, of a milk, of a gel, of an ointment, or of a foaming composition. These excipients are compounds commonly used in the formulation of a pharmaceutical composition. Preferably, the active agent-solubilizing excipients according to the invention are water, alcohols, polyols, ethers, esters, aldehydes, ketones, fatty acids and fatty alcohols, and fatty esters. More preferably, the excipient used will be an alcohol. According to the invention, the term "alcohol" is intended to mean linear or branched aliphatic alcohols such as ethanol, propanol or isopropanol.

In a preferred embodiment according to the invention, the vehicle used will therefore be alcoholic.

According to the invention, the term "alcoholic vehicle" is intended to mean a vehicle comprising at least 15% of alcohol, and preferably at least 25% of ethanol.

The pharmaceutical composition according to the invention may also contain inert additives or combinations of these additives, such as:
- wetting agents;
- flavour enhancers;
- preserving agents;
- stabilizers;
- moisture regulators;
- pH regulators;
- osmotic pressure modifiers;
- emulsifiers;
- UV-A and UV-B screening agents;
- propenetrating agents;
- antioxidants;
- and synthetic polymers.

Of course, those skilled in the art will take care to choose the possible compound(s) to be added to these compositions in such a way that the advantageous properties intrinsically associated with the present invention are not, or are not substantially, altered by the envisaged addition.

The composition according to the invention is more particularly intended for treating the skin and the mucous membranes, and may be provided in the form of ointments, creams, milks, salves, powders, impregnated pads, syndets, solutions, gels, sprays, foams, suspensions, lotions, sticks, shampoos, pledgets or washing bases. It may also be provided in the form of suspensions of lipid or polymer vesicles or nanospheres or microspheres or polymer patches and hydrogels to allow controlled release. This topical-application composition may be provided in anhydrous form, in aqueous form or in the form of an emulsion.

The composition according to the invention showing improved penetration is preferably administered in the form of a sprayable composition. In order to be sprayable, the compositions according to the invention will preferably have a viscosity of less than 50 centistokes, and more preferably less than 10 centistokes.

The sprayable form, or spray, can be obtained by conventional formulation means known to those skilled in the art. For example, the composition may be sprayed by means of a mechanical spraying device which pumps the composition from a container, bottle or equivalent. The composition passes through a nozzle which can be aimed directly at the desired site of application. The nozzle can be chosen so as to apply the composition in the form of a vaporization or of a jet of droplets, according to techniques known to those skilled in the art. According to the pharmaceutical active agent chosen, the spraying mechanism must be capable of always delivering the same amount of active agent. The mechanisms for controlling the amount of composition to be delivered by the spray are also known to those skilled in the art.

Preferably, for the composition according to the invention, a dosing spray bottle, for which the application area and dose characteristics are controlled and reproducible, will be used. For example, the spray device used consists of a bottle equipped with a 25 µl dosing valve.

A subject of the present invention is also the use of a composition according to the invention, for producing a medicinal product intended for treating:
- dermatological conditions associated with a keratinization disorder relating to differentiation and to proliferation, in particular common acne, comedo-type acne, polymorphic acne, rosacea, nodulocystic acne, acne conglobata, senile acne, and secondary acne such as solar, drug-related or occupational acne,
- ichthyoses, ichthyosiform conditions, Darrier's disease, palmoplantar keratoderma, leukoplakia and leukoplakiform conditions, and cutaneous or mucosal (oral) lichen,
- dermatological conditions with an inflammatory immunoallergic component, with or without a cell proliferation disorder, in particular cutaneous, mucosal or ungual psoriasis, psoriatic rheumatism, cutaneous atopy, such as eczema, respiratory atopy or gingival hypertrophy,
- benign or malignant dermal or epidermal proliferations, of viral or nonviral origin, in particular common warts, flat warts, epidermodysplasia verruciformis, oral or florid papillomatoses, and T lymphoma,
- proliferations which may be induced by ultraviolet light, in particular basal cell epithelioma and spinocellular epithelioma,
- precancerous skin lesions, in particular keratoacanthomas,
- immune dermatoses, in particular lupus erythematous,
- bullous immune diseases,
- collagen diseases, in particular scleroderma,
- dermatological or systemic conditions with an immunological component,
- skin disorders due to exposure to UV radiation, or light-induced or chronological ageing of the skin, or actinic keratoses and pigmentations, or any pathologies associated with chronological or actinic ageing, in particular xerosis,
- sebaceous function disorders, in particular hyperseborrhoea acne or simple seborrhoea or seborrhoeic dermatitis,
- cicatrization disorders or stretch marks,
- pigmentation disorders, such as hyperpigmentation, melasma, hypopigmentation or vitiligo,
- lipid metabolism conditions, such as obesity, hyperlipidemia, non-insulin-dependant diabetes or syndrome X,
- inflammatory conditions such as arthritis,
- cancerous or precancerous states,
- alopecia of various origins, in particular alopecia caused by chemotherapy or radiation,
- immune system disorders, such as asthma, type 1 diabetes mellitus, multiple sclerosis or other selective dysfunctions of the immune system, or
- cardiovascular system conditions such as arteriosclerosis or hypertension.

In a preferred embodiment of use of the composition, said composition will contain 0.3% of (4E,6E)-7-[3-(3,4-bishydroxymethylbenzyloxy)phenyl]-3-ethylnona-4,6-dien-3-ol and will be used for producing a medicinal product intended to treat psoriasis.

The invention also relates to a process for improving the penetration of an active agent derived from vitamin D, characterized in that a composition comprising the following, in a pharmaceutically acceptable vehicle, is applied to the skin:
a) a therapeutically effective amount of an active agent derived from vitamin D,
b) at least one volatile silicone,
c) a nonvolatile oily phase,
said composition being applied in the form of a spray.

In fact, the applicant has discovered, surprisingly, that the penetration of an active agent, and in particular of compounds derived from vitamin D, through the skin is improved by the composition according to the invention, even in the absence of excipients with propenetrating activity.

The expression "improvement in penetration into the skin" is intended to mean a significant increase in penetration into the skin of at least a factor of 2, compared to formulations previously produced on the market.

The penetration of the active agent is measured according to the protocol described in Example 4.

The following examples show, in a nonexhaustive manner, examples of formulation of the composition according to the invention and results of penetration into the skin.

### Example 1:

The formulation is obtained by mixing the various compounds mentioned below until a homogeneous and clear solution is obtained.

| Ingredients | Function | Spray A |
|---|---|---|
| (4E,6E)-7-[3-(3,4-bis-hydroxymethylbenzyloxy)phenyl]-3-ethylnona-4,6-dien-3-ol | Active agent | 0.3% |
| Hexamethyldisiloxane | Volatile silicone | 60.0% |
| Paraffin oil | Nonvolatile oily phase | 10.0% |
| Absolute ethanol | Solvent: excipient | qs 100% |

### Example 2:

The procedure used is the same as that in Example 1.

| Ingredients | Function | Spray B |
|---|---|---|
| (4E,6E)-7-[3-(3,4-bis-hydroxymethylbenzyloxy)phenyl]-3-ethylnona-4,6-dien-3-ol | Active agent | 0.3% |
| Hexamethyldisiloxane | Volatile silicone | 59.4% |
| Silicone gum | Silicone gum | 0.6% |
| Paraffin oil | Nonvolatile oily phase | 10.0% |
| Absolute ethanol | Solvent | qs 100% |

### Example 3:

The procedure used is the same as that in Example 1.

| Ingredients | Function | Spray C |
|---|---|---|
| (4E,6E)-7-[3-(3,4-bis-hydroxymethylbenzyloxy)phenyl]-3-ethylnona-4,6-dien-3-ol | Active agent | 0.3% |
| Hexamethyldisiloxane | Volatile silicone | 59.4% |
| Silicone gum | Silicone gum | 0.6% |
| Paraffin oil | Nonvolatile oily phase | 10.0% |
| Oleic acid | Propenetrating agent | 5.0% |
| Butylhydroxytoluene (BHT) | Antioxidant | 0.05% |
| Absolute ethanol | Solvent | qs 100% |

Example 4: Study of the release/penetration *in vitro,* on human skin, of (4E,6E)-7-[3-(3,4-bishydroxymethylbenzyloxy)phenyl]-3-ethylnona-4,6-dien-3-ol contained in two different formulations, one of which is sprayable according to the preferred embodiment of the invention.

The first aim is to quantify the penetration into the skin of the active agent formulated in both formulations, *in vitro,* on human skin, after 16 hours of application. A sprayable formula according to the invention is compared with a composition in the form of an ointment.

The exact compositions of the two formulations are given in Table 1 below.

**TABLE 1**

| Ingredients | Function | Spray (in %) | Ointment (in %) |
|---|---|---|---|
| (4E,6E)-7-[3-(3,4-bishydroxymethylbenzyloxy)-phenyl]-3-ethylnona-4,6-dien-3-ol | Active agent | 0.3 | 0.3 |
| Hexamethyldisiloxane | Volatile silicone | 59.40 | |
| Silicone gum | Silicone gum | 0.6 | |
| Paraffin oil | Occlusive agent | 10.00 | 5 |
| Oleic acid | Propentrating agent | / | / |
| Propylene glycol | Propentrating agent | | 10.00 |
| White petroleum jelly | Occlusive agent | | 76.94 |
| Macrogol 2 stearyl ether | | | 5 |
| Disodium EDTA | Chelating agent | | 0.0065 |
| DL-alpha-tocopherol | Antioxidant | | 0.12 |
| BHT | Antioxidant | | |
| Absolute ethanol | Solvent | qs 100% | |
| Water | Solvent | | qs 100% |

**Experimental conditions:** Percutaneous absorption is evaluated by means of diffusion cells consisting of 2 compartments separated by human skin. The formulations were applied without occlusion for 16 hours. The formulations were applied at a rate of 10 mg of formulation per cm² (i.e. 30 micrograms of (4E,6E)-7-[3-(3,4-bishydroxymethylbenzyloxy)phenyl]-3-ethylnona-4,6-dien-3-ol). Throughout the duration of the study, the dermis is in contact with a recipient liquid which is not renewed as a function of time (static mode). At the end of the application period, the surface excess is removed and the distribution of the (4E,6E)-7-[3-(3,4-bishydroxymethylbenzyloxy)phenyl]-3-ethylnona-4,6-dien-3-ol is quantified in the various skin compartments and in the recipient liquid. The concentrations of (4E,6E)-7-[3-(3,4-bishydroxymethylbenzyloxy)phenyl]-3-ethylnona-4,6-dien-3-ol were quantified using an HPLC/MS/MS method conventionally known to those skilled in the art (LQ: 10 ng.mL⁻¹). The spray formula was applied using a spray bottle equipped with a 25 µl dosing valve.

The experimental results show that, whichever the formulation tested, the active agent is distributed mainly in the skin (epidermis, including strateum corneum, and dermis). The total amounts penetrated (stratum corneum + epidermis + dermis + recipient liquid) are:

| | | Application time: 16 hours |
|---|---|---|
| Spray | | |
| Total amount having penetrated | | 2.64 ± 0.50 µg |
| | - µg | |
| | - % dose applied | 9.2% |
| Ointment | | |
| Total amount having penetrated | | |
| | - µg | 0.63 ± 0.14 µg |
| | - % dose applied | 2.3% |

### Results:

The results show here that the spray formula according to the invention shows a four-fold increase in penetration of the active agent after 16 hours, compared with the ointment formula.

This result therefore indicates that the compositions according to the invention make it possible to obtain a significant improvement in penetration of an active agent derived from vitamin D compared with existing formulas.

The spray formulas as described therefore make it possible to do without the use of glycols, without decreasing skin penetration, and therefore show an additional advantage in terms of nonirritant potential versus the compositions comprising a high content of glycol.

## Claims

1. Composition comprising, in a pharmaceutically acceptable vehicle:
a) a therapeutically effective amount of at least one pharmaceutical active agent,
b) at least one volatile silicone,
c) a nonvolatile oily phase,
**characterized in that** the pharmaceutical active agent is calcitriol or 1,25-dihydroxy vitamin D₃ and **in that** the composition is sprayable.

2. Composition according to Claim 1,
**characterized in that** it comprises:
a) between 0.0001 and 20% by weight of the active agent,
b) between 25 and 95% by weight of volatile silicone,
c) between 1 and 50% by weight of nonvolatile oily phase.

3. Composition according to Claim 1 or 2, **characterized in that** the vehicle is a solvent of the pharmaceutical active agent.

4. Composition according to one of the Claims 1 to 3, **characterized in that** the vehicle comprises an ester.

5. Composition according to one of the Claims 1 to 3, **characterized in that** the vehicle comprises a fatty ester.

6. Composition according to one of Claims 1 to 5, **characterized in that** the volatile silicone is chosen from the group consisting of polydimethylcyclosiloxanes.

7. Composition according to one of Claims 1 to 6, **characterized in that** the nonvolatile oily phase is chosen from the group consisting of glyceryl esters of fatty acid, esters, non-volatile hydrocarbons and silicone oils, or their mixtures.

8. Composition according to one of Claims 1 to 7, **characterized in that** nonvolatile oily phase is mineral oil.

9. Composition according to one of Claims 1 to 8, **characterized in that** nonvolatile oily phase is caprylic/ capric triglycerides.

10. Composition according to one of Claims 1 to 9, **characterized in that** it contains between 1 and 50% of the nonvolatile oily phase.

11. Composition according to any one of the claims 1 to 10, **characterized in that** it comprises:
a) between 0.0001 and 20% by weight of calcitriol,
b) between 25 and 95% by weight of polydimethylcyclosiloxanes,
c) between 1 and 50% by weight of nonvolatile oily phase, chosen from the group consisting of glyceryl esters of fatty acid, esters, non-volatile hydrocarbons and silicone oils, or their mixtures
d) an active agent-solubilizing vehicle comprising an ester.

12. Composition according to any one of the claims 1 to 11, **characterized in that** it comprises:
a) between 0.0001 and 0,5% by weight of the active agent,
b) between 25 and 95% by weight of polydimethylcyclosiloxanes,
c) between 7 and 50% by weight of nonvolatile oily phase, chosen from the group consisting of glyceryl esters of fatty acid, esters, non-volatile hydrocarbons and silicone oils, or their mixtures,
d) an active agent-solubilizing vehicle comprising an ester.

13. Use of a composition according to any one of Claims 1 to 12, for producing a medicinal product intended for treating:
- dermatological conditions associated with a keratinization disorder relating to differentiation and to proliferation, in particular common acne, comedo-type acne, polymorphic acne, rosacea, nodulocystic acne, acne conglobata, senile acne, and secondary acne such as solar, drug-related or occupational acne,
- ichthyoses, ichthyosiform conditions, Darrier's disease, palmoplantar keratoderma, leukoplakia and leukoplakiform conditions, and cutaneous or mucosal (oral) lichen,
- dermatological conditions with an inflammatory immunoallergic component, with or without a cell proliferation disorder, in particular cutaneous, mucosal or ungual psoriasis, psoriatic rheumatism, cutaneous atopy, such as eczema, respiratory atopy or gingival hypertrophy,
- benign or malignant dermal or epidermal proliferations, of viral or nonviral origin, in particular common warts, flat warts, epidermodysplasia verruciformis, oral or florid papillomatoses, and T lymphoma,
- proliferations which may be induced by ultraviolet light, in particular basal cell epithelioma and spinocellular epithelioma,
- precancerous skin lesions, in particular keratoacanthomas,
- immune dermatoses, in particular lupus erythematous,
- bullous immune diseases,
- collagen diseases, in particular scleroderma,
- dermatological or systemic conditions with an immunological component,
- skin disorders due to exposure to UV radiation, or light-induced or chronological ageing of the skin, or actinic keratoses and pigmentations, or any pathologies associated with chronological or actinic ageing, in particular xerosis,
- sebaceous function disorders, in particular hyperseborrhoea acne or simple seborrhoea or seborrhoeic dermatitis,
- cicatrization disorders or stretch marks,
- pigmentation disorders, such as hyperpigmentation, melasma, hypopigmentation or vitiligo.

14. Use of a composition, according to Claim 13, for treating psoriasis or cutaneous atopy.

15. Process for improving the penetration of calcitriol, **characterized in that** a composition comprising the following, in a pharmaceutically acceptable vehicle is applied to the skin:
a) a therapeutically effective amount of calcitriol,
b) at least one volatile silicone,
c) a nonvolatile oily phase,
said composition being applied in the form of a spray.

16. Process according to Claim 15, **characterized in that** the volatile silicone is polydimethylcyclosiloxanes, and the nonvolatile oily phase is chosen from the group consisting of glyceryl esters of fatty acid, esters, non-volatile hydrocarbons and silicone oils, or their mixtures.
